# EUROPEAN PATENT APPLICATION

(11) **EP 4 582 438 A1**
(43) Date of publication of application: **09.07.2025**
(21) Application number: 23860908.5
(22) Date of filing: 31.08.2023
(51) Int. Cl.: C07K 14/00, A01N 37/46

(54) **ANTIBACTERIAL PEPTIDE CONSISTING OF TRANSMEMBRANE PEPTIDE DOMAIN AND BASIC OR POLAR AMINO ACIDS AT OPPOSITE ENDS THEREOF AND VARIANTS THEREOF**

(30) Priority: 01.09.2022 KR 20220110830; 30.08.2023 KR 20230114740
(71) Applicant: Korea University Research and Business Foundation, Seoul 02841 (KR)
(72) Inventor: LEE, So Ra, Seoul 08844 (KR); YOO, Young Do, Seoul 06284 (KR)
(74) Representative: Isarpatent
(86) International application number: PCT/KR2023/012962
(87) International publication number: WO 2024/049232

(57) **Abstract**

The present disclosure relates to a fusion polypeptide comprising a polypeptide containing a transmembrane domain (TMD) having the C-terminus and the N-terminus to which a polypeptide of general formula I is bound, and the fusion polypeptide can provide antibacterial activity against Gram-positive, Gram-negative, and multidrug-resistant bacteria.

## Description

### [Technical Field]

The present disclosure relates to an antibacterial peptide including a transmembrane peptide domain (transmembrane domain) in a membrane protein located in a cell membrane and an intracellular organelle membrane in a cell and basic or polar (uncharged) amino acids at opposite ends of the domain, and more particularly, to provide peptides and antibacterial compositions including the peptides by confirming antibacterial activity of peptides existing in nature and various artificially synthesized peptides having these characteristics against Gram-positive, Gram-negative, and multidrug-resistant bacteria.

### [Background Art]

The biggest problem with antibiotics used as a treatment for bacterial infections is the acquisition of antibiotic resistance to bacteria. According to a report by the Wellcome Trust of the British government, by 2050, the number of deaths worldwide due to superbacteria that may not be treated with conventional antibiotics exceed 10 million, which is expected to make superbacteria infections a threat that will overtake cancer as a cause of death.

In addition, the World Health Organization (WHO) has selected carbapenem-resistant *Acinetobacter baumannii,* carbapenem-resistant *Pseudomonas aeruginosa,* and carbapenem-resistant *Enterobacteriaceae sp.* as bacteria of critical priority among numerous multidrug-resistant bacteria and announced that the top priority should be given to solving the problem of Gram-negative superbacteria that are resistant to carbapenem antibiotics, known as last-line antibiotics.

As mentioned above, the biggest problem with antibiotics that have been used by mankind to date is the acquisition of bacterial resistance, and much research is being conducted to solve this problem, and particularly, antibacterial peptides (AMPs) is being actively developed to overcome the acquisition of antibiotic resistance to bacteria. Various antibacterial peptides derived from animal or human host defense proteins have been developed, but have not been put into practical use due to problems such as the possibility of acquiring bacterial resistance to the human immune system, short half-life in the blood, toxicity to the human body, and limited efficacy compared to conventional antibiotics. For example, magainin, an antibacterial peptide developed in the United States in 1999, was requested to receive FDA approval after a phase 3 clinical trial, but was rejected because its efficacy was not superior to that of conventional antibiotics. In addition, daptomycin in 2003 and oritavancin in 2014 were approved by the US Food and Drug Administration, but both peptides showed antibacterial activity only against Gram-positive bacteria and may be applied only to skin infections.

To solve the problem, the present inventors confirmed in the prior art that a peptide (KU-5878, AMPR-11) containing the second transmembrane domain of a Romo1 protein located in the inner mitochondrial membrane exhibited antibacterial activity against Gram-positive, Gram-negative, and multidrug-resistant bacteria, and thus could be applied to the treatment of a wide range of bacterial infectious diseases, and applied for a patent and published a paper. However, in the case of KU-5878, although the transmembrane peptide domain was included, its antibacterial activity was not excellent because one basic amino acid was included in each of opposite ends of the domain. The present inventors have discovered, through further advanced research, that a peptide consisting of a transmembrane peptide domain (transmembrane domain) in a membrane protein and a total of three or more basic amino acids and some polar (uncharged) amino acids at opposite ends of the domain had better antibacterial activity than the prior art, and confirmed that the peptide may be applied to the treatment of a wide range of bacterial infectious diseases by exhibiting antibacterial activity against Gram-positive, Gram-negative, and multidrug-resistant bacteria, and then completed the present disclosure.

### [Disclosure]

### [Technical Problem]

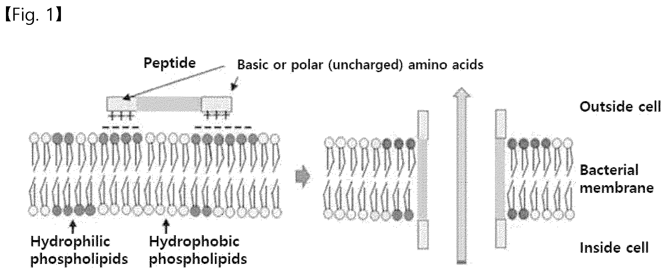

An object to be achieved by the present disclosure is to provide a fusion polypeptide including a polypeptide including a transmembrane domain (TMD) consisting of 15 to 30 amino acids, which is an antibacterial peptide consisting of a transmembrane peptide domain (transmembrane domain) in a cell and a total of three or more basic amino acids and some polar amino acids at opposite ends of the domain (FIG. 1), and terminal amino acids including one or more amino acid bound to each of the C-terminus and the N-terminus of the polypeptide or a total of three or more amino acids bound to both termini.

Further, another object of the present disclosure is to provide an antibiotic including the antibacterial peptide as an active ingredient, and food and feed additives, a cosmetic composition, a biopesticide, and an antibacterial quasi-drug composition including the antibacterial peptide.

However, objects of the present disclosure are not limited to the aforementioned objects, and other objects which are not mentioned may be clearly understood to those skilled in the art from the following description.

### [Technical Solution]

An aspect of the present disclosure provides a fusion polypeptide including polypeptide including a transmembrane domain (TMD) consisting of 15 to 30 amino acids and terminal amino acids including one or more amino acid bound to each of the C-terminus and the N-terminus of the polypeptide or a total of three or more amino acids bound to both termini.

Another aspect of the present disclosure provides an antibacterial polypeptide including the fusion polypeptide.

Yet another aspect of the present disclosure provides an antibiotic, food and feed additives, a cosmetic composition, a biopesticide, and an antibacterial quasi-drug composition including the polypeptide.

### [Advantageous Effects]

According to the present disclosure, the peptide and variants thereof have higher antibiotic activity against various types of bacteria than conventional antibiotics and peptides in the prior art. In particular, the peptide of the present disclosure exhibits high antibacterial activity against bacteria resistant to antibiotics to overcome the limitations of the application of conventional antibiotics and thus can be provided as an agent for preventing or treating infectious diseases caused by multidrug-resistant bacteria. Therefore, it is expected that the antibacterial peptide will be used in various ways, such as pharmaceutical use, external pharmaceutical use, food and feed additives, pesticides, and cosmetics additives, for the purpose of preventing or treating a wide range of bacterial infectious diseases.

### [Description of Drawings]

FIG. 1 is a diagram of an antibacterial peptide consisting of a transmembrane domain (TMD) and basic or polar (uncharged) amino acids at opposite ends of the domain. The diagram illustrates that the peptide forms pores in the cell membrane of Gram-positive, Gram-negative and multi-drug resistant bacteria to kill the bacteria.

### [Best Mode]

An aspect of the present disclosure provides a fusion polypeptide including a polypeptide including a transmembrane domain (TMD) consisting of 15 to 30 amino acids and terminal amino acids including one or more, a total of three or more amino acid bound to each of the C-terminus and the N-terminus of the polypeptide.

In one specific example of the present disclosure, the transmembrane domain (TMD) may be any one of SEQ ID NOs: 39, 41, 54, and 73 to 78.

In one specific example of the present disclosure, the terminal amino acid may have 2 to 6 amino acids bound to each of the C-terminus and the N-terminus.

In one specific example of the present disclosure, the terminal amino acid may be selected from the group consisting of basic amino acids and polar amino acids.

In one specific example of the present disclosure, the terminal amino acid may be any one of lysine, arginine, histidine, serine, threonine cysteine, glutamine, asparagine and tyrosine.

In one specific example of the present disclosure, the fusion polypeptide may be any one of SEQ ID NOs: 43, 44, 45, 46, 47, 48, 49, 50, 51, 52, 53, 56, 57, 59, 61, 63, 64, 65, 66, 71 and 72.

Another aspect of the present disclosure provides an antibacterial polypeptide including the fusion polypeptide.

In one specific example of the present disclosure, the polypeptide may further include PEGylation, acetylation, carboxylation, lipidation, or amidation at the N-terminus or C-terminus.

In one specific example of the present disclosure, the polypeptide may have antibacterial activity against one or more bacteria selected from the group consisting of Gram-positive bacteria, Gram-negative bacteria, and multidrug-resistant bacteria.

Yet another aspect of the present disclosure provides an antibiotic, a food additive, a feed additive, a cosmetic composition, an antibacterial biopesticide preparation and an antibacterial quasi-drug composition including the polypeptide as an active ingredient.

### [Modes]

An aspect of the present disclosure provides a fusion polypeptide including a polypeptide including a transmembrane domain (TMD) consisting of 15 to 30 amino acids and terminal amino acids including one or more amino acid bound to each of the C-terminus and the N-terminus of the polypeptide or a total of three or more amino acids bound to both termini.

The transmembrane domain is a peptide sequence region having membrane penetration ability, and a peptide having membrane penetration ability is a peptide that exists by penetrating the lipid bilayer of an organelle existing within a cell. In the transmembrane peptide, the transmembrane domain (TMD) that penetrates the lipid layer consists of about 22 amino acids, which consist of hydrophobic amino acids and some hydrophilic amino acids. One or several transmembrane domains may be included.

A significant portion of a genome corresponds to transmembrane proteins, and transmembrane portions may be predicted with relative accuracy through computer algorithms. In the present disclosure, transmembrane protein sequence information was obtained from UniProt (www.uniprot.org).

The present inventors synthesized peptides of various lengths consisting of a transmembrane domain and a total of three or more basic amino acids and some polar amino acids at opposite ends of the domain based on the information obtained by searching in UniProt, confirmed antibacterial activity thereof, confirmed antibacterial activity from variants of the peptides, and then completed the present disclosure (see Examples 1 and 2).

In a specific example of the present disclosure, the present inventors synthesized peptides of various lengths existing in nature, which consist of a transmembrane domain among human proteins and a total of three or more basic amino acids and polar amino acids at opposite ends of the domain, as described above, and confirmed antibacterial activity of each peptide against *Pseudomonas aeruginosa* and *Staphylococcus aureus* using a minimum bactericidal concentration measurement method. As a result, it was confirmed that all of the synthesized peptides had better antibacterial activity than the previously invented KU-5878 (see Example 2).

Furthermore, when a peptide was synthesized in which opposite ends of the peptide represented by SEQ ID NOs: 1 and 2 of the present disclosure were partially deficient and the antibacterial activity was confirmed, it was confirmed that the antibacterial activity was reduced. Since it was confirmed that a peptide without basic or polar amino acids at opposite ends of the transmembrane domain represented by 39 and 41 had no antibacterial activity, it was confirmed that the basic or polar amino acid sequences at opposite ends of the transmembrane domain were important for the antibacterial activity (see Example 3).

In another specific example of the present disclosure, the present inventors confirmed the antibacterial activity of a synthetic peptide in which basic amino acids are artificially repeatedly attached to opposite ends of the transmembrane domain. As a result, it was found that a group of peptides synthesized by attaching a total of three or more lysines (K) or arginines (R) to opposite ends of the transmembrane domain exhibited excellent antibacterial activity (see Example 4).

In a specific example of the present disclosure, the present inventors confirmed the antibacterial activity of the fusion polypeptide of the present disclosure against *Enterococcus faecium, Escherichia coli, Acinetobacter baumannii,* multidrug-resistant *Pseudomonas aeruginosa,* multidrug-resistant *Staphylococcus aureus,* multidrug-resistant *Enterococcus faecium* and multidrug-resistant *Acinetobacter baumannii.* As a result, it was found that all representative peptides exhibited a wide range of antibacterial activities against Gram-positive bacteria, Gram-negative bacteria, and multidrug-resistant bacteria (see Examples 5 and 6).

In addition, in another specific example of the present disclosure, the present inventors synthesized a peptide consisting of a transmembrane domain of a species other than human and a total of three or more basic amino acids and polar amino acids at opposite ends of the domain, and confirmed the antibacterial activity, and as a result, it was found that the peptide having common characteristics exhibited antibacterial activity regardless of the species (see Example 7).

In addition, in another specific example of the present disclosure, the present inventors confirmed that the amino acid sequences bound to the opposite ends exhibited excellent antibacterial activity even when bound to other transmembrane peptides (see Example 8).

In the present disclosure, the term "peptide" refers to a linear molecule formed by binding amino acid residues with each other by peptide bonds. The peptide may be prepared by a chemical synthesis method known in the art, particularly a solid-phase synthesis technique or a liquid-phase synthesis technique.

In one specific example of the present disclosure, the transmembrane domain may consist of 17 to 25 amino acids, and more specifically, any one of SEQ ID NOs: 39, 41, 54, and 73 to 78.

The terminal amino acids are bound to the C-terminus and the N-terminus of the transmembrane peptide domain, and each terminus includes one or more amino acids, a total of three or more amino acids, specifically, each of the C-terminus and the N-terminus includes 3 to 6 amino acids, so that at least 1 and at most 6 amino acids may be bound to both termini.

The terminal amino acid may be selected from basic amino acids and polar amino acids, and the terminal amino acid may be any one of lysine, arginine, histidine, serine, threonine cysteine, glutamine, asparagine, and tyrosine.

At this time, it is sufficient that the terminal amino acids bound to the C-terminus and the N-terminus are one or more at each of the both termini and the sum of the number of amino acids at the both termini is 3 or more, and each amino acid is independent of each other, and the peptides bound to the C-terminus and the N-terminus have different amino acid components and different lengths, which is also included in the present disclosure. As an example of the terminal amino acid, it may be configured such that one amino acid is bound to one terminus of the transmembrane domain and two or more amino acids are bound to the other terminus.

In one specific example of the present disclosure, the fusion polypeptide may be any one of SEQ ID NOs: 43, 44, 45, 46, 47, 48, 49, 50, 51, 52, 53, 56, 57, 59, 61, 63, 64, 65, 66, 71 and 72.

As yet another embodiment of the present disclosure, the polypeptide may be PEGylated, acetylated, carboxylated, lipidated, or amidated. More particularly, the amino terminus may be bound with a protecting group, such as an acetyl group, a fluorenyl methoxycarbonyl group, a formyl group, a palmitoyl group, a myristyl group, a stearyl group, and polyethylene glycol (PEG), and the carboxy terminus of the peptide may be modified with a hydroxyl group (-OH), an amino group (-NH₂), an azide (-NHNH₂), or the like. In addition, the terminus of the peptide of the present disclosure or an R-residue (R-group) of the amino acid may be bound with fatty acids, oligosaccharide chains, all nanoparticles (gold particles, liposomes, heparin, hydrogel, etc.), amino acids, carrier proteins, and the like.

As yet another embodiment of the present disclosure, the amino acids constituting the peptide may each independently be L-form or D-form amino acids and may be radioactive or fluorescently labeled amino acid analogues.

In yet another embodiment of the present disclosure, the peptide may have antibacterial activity against one or more bacteria selected from the group consisting of Gram-positive bacteria, Gram-negative bacteria, and multidrug-resistant bacteria.

Another aspect of the present disclosure provides an antibacterial polypeptide including the fusion polypeptide.

As described above, the fusion polypeptide may have an antibacterial effect by penetrating the cell membrane and dissolving bacteria by further including a transmembrane peptide domain and amino acid residues at opposite ends of the domain, and thus the present disclosure may provide a polypeptide having an antibacterial effect including the fusion polypeptide.

In a specific example of the present disclosure, the polypeptide may further include PEGylation, acetylation, carboxylation, lipidation, or amidation at the N-terminus or C-terminus. More particularly, the amino terminus may be bound with a protecting group, such as an acetyl group, a fluorenyl methoxycarbonyl group, a formyl group, a palmitoyl group, a myristyl group, a stearyl group, and polyethylene glycol (PEG), and the carboxy terminus of the peptide may be modified with a hydroxyl group (-OH), an amino group (-NH₂), an azide (-NHNH₂), or the like. In addition, the terminus of the peptide of the present disclosure or an R-residue (R-group) of the amino acid may be bound with fatty acids, oligosaccharide chains, all nanoparticles (gold particles, liposomes, heparin, hydrogel, etc.), amino acids, carrier proteins, and the like.

In one specific example of the present disclosure, the polypeptide may have antibacterial activity against one or more bacteria selected from the group consisting of Gram-positive bacteria, Gram-negative bacteria, and multidrug-resistant bacteria.

In yet another embodiment of the present disclosure, the Gram-positive bacteria may be bacteria belonging to a genus selected from the group consisting of *Staphylococcus sp., Bacillus sp., Enterococcus sp., Streptomyces sp.,* and *Streptococcus sp.* Preferably, the Gram-positive bacteria may be one or more bacteria selected from the group consisting of *Staphylococcus aureus, Bacillus subtilis, Enterococcus faecium, Streptomyces sindenensis, Enterococcus faecalis,* and *Streptococcus pneumoniae.*

In yet another embodiment of the present disclosure, the Gram-negative bacteria may be bacteria belonging to a genus selected from the group consisting of *Escherichia sp., Klebsiella sp.,Acinetobacter sp., Pseudomonas sp.,* and *Enterobacter sp.* Preferably, the Gram-negative bacteria may be one or more bacteria selected from the group consisting of *Escherichia coli, Klebsiella pneumoniae, Acinetobacter baumannii, Pseudomonas aeruginosa,* and *Enterobacter aerogenes.*

In yet another embodiment of the present disclosure, the multidrug-resistant bacteria may be the Gram-positive bacteria or Gram-negative bacteria having resistance to one or more antibiotics belonging to penicillins, carbapenems, cephalosporins, quinolones, macrolides, tetracyclins, or glycopeptides. Preferably, the multidrug-resistant bacteria may be bacteria belonging to a genus selected from the group consisting of methicillin-resistant *Staphylococcus sp.,* multidrug-resistant *Pseudomonas sp.,* vancomycin-resistant *Enterococcus sp.,* multidrug-resistant *Klebsiella sp.,* or multidrug-resistant *Acinetobacter sp.,* and vancomycin-resistant *Staphylococcus sp.*

In yet another embodiment of the present disclosure, the multidrug-resistant bacteria may be one or more bacteria selected from the group consisting of methicillin-resistant *S. aureus,* multidrug-resistant *P. aeruginosa,* multidrug-resistant *A. baumannii,* multidrug-resistant *K. pneumoniae,* vancomycin-resistant *E. faecium,* and vancomycin-resistant *S. aureus.*

In yet another embodiment of the present disclosure, the multidrug-resistant *P. aeruginosa* may have resistance to one or more antibiotics selected from the group consisting of piperacillin, piperacilin-tazobactam, ceftazidime, imipenem, meropenem, gentamicin, amikacin, and ciprofloxacin. The multidrug-resistant *A. baumannii* may have resistance to one or more antibiotics selected from the group consisting of piperacillin, piperacilin-tazobactam, ceftazidime, imipenem, meropenem, gentamicin, amikacin, ciprofloxacin, and cefepime. The multidrug-resistant *K. pneumoniae* may have resistance to one or more antibiotics selected from the group consisting of piperacillin tazobactam, ceftazidime, cefepime, imipenem, gentamicin, and ciprofloxacin.

In yet another embodiment of the present disclosure, the vancomycin-resistant *E*. *faecium* may further have resistance to one or more antibiotics selected from the group consisting of rifampin, tetracycline, gentamicin, erythromycin, streptomycin, and ampicillin in addition to vancomycin, and the vancomycin-resistant *S. aureus* may further have resistance to one or more antibiotics selected from the group consisting of oxacillin, benzylpenicillin, ampicillin, and cefazolin in addition to vancomycin.

Another aspect of the present disclosure provides an antibiotic including the polypeptide as an active ingredient.

In addition, the present disclosure provides a pharmaceutical composition for preventing or treating bacterial infectious diseases, including the antibacterial peptide as an active ingredient.

In the present disclosure, "prevention" means all actions that delay infection by the Gram-positive bacteria, Gram-negative bacteria, and/or multidrug-resistant bacteria, or delay the onset of diseases caused by the infection, by administering the pharmaceutical composition according to the present disclosure. The "treatment" refers to all actions that improve or beneficially change symptoms caused by the bacterial infection by administering the pharmaceutical composition according to the present disclosure.

In the present disclosure, the pharmaceutical composition may further include one or more known antibiotics in addition to the fusion polypeptide or antibacterial polypeptide, and may further include appropriate carriers, excipients, and diluents commonly used in the preparation of the pharmaceutical composition.

In the present disclosure, the "carrier" is also called a vehicle, and refers to a compound that facilitates the addition of a protein or peptide into a cell or tissue, and for example, dimethylsulfoxide (DMSO) is a commonly used carrier that facilitates the injection of many organic substances into the cell or tissue of a living organism.

In the present disclosure, the "diluent" is defined as a compound diluted in water that not only stabilizes a biologically active form of a target protein or peptide, but also dissolves the protein or peptide. Salts dissolved in a buffer solution are used as diluents in the art. A commonly used buffer solution is phosphate buffered saline because the phosphate buffered saline imitates a salt state of a human solution. Since the buffer salt may control the pH of the solution at a low concentration, the buffer diluent rarely modifies the biological activity of the compound. Compounds containing azelaic acid used herein may be administered to human patients by themselves or as a pharmaceutical composition mixed with other ingredients or with suitable carriers or excipients, like combination therapy.

In addition, the pharmaceutical composition according to the present disclosure may be formulated and used in the form of powders, granules, tablets, capsules, suspensions, emulsions, syrups, external preparations such as aerosols, and sterile injectable solutions according to each conventional method. The pharmaceutical composition of the present disclosure may be administered orally or parenterally (e.g., applied intravenously, subcutaneously, intraperitoneally, or topically) depending on an intended method. The dose varies depending on the condition and weight of a patient, the degree of a disease, a drug form, and administration route and period, but may be appropriately selected by those skilled in the art. For example, the dose may be administered in 0.001 mg to 1000 mg in the form mixed with the pharmaceutically acceptable carrier. The pharmaceutical composition of the present disclosure may be administered once or several times a day as needed, and may be used alone or in combination with surgery, hormone therapy, drug therapy, and methods using biological response modifiers.

Further, the present disclosure provides a method for preventing or treating bacterial infectious disease including administering the antibacterial peptide to a subject.

Further, the present disclosure provides a use of the antibacterial peptide for the preparation of a drug for the prevention or treatment of bacterial infectious disease.

In one embodiment of the present disclosure, the bacterial infectious disease may be at least one disease selected from the group consisting of skin infection, food poisoning, otitis media, cystitis, peritonitis, urinary tract infection, mastitis, pneumonia, endocarditis, conjunctivitis, arthritis, endometritis, strangles, bacteremia, sepsis, osteomyelitis, and acne, and preferably pneumonia or sepsis.

Yet another aspect of the present disclosure provides a food additive, a feed additive, a cosmetic composition, an antibacterial biopesticide preparation and an antibacterial quasi-drug composition including the polypeptide.

When the peptide of the present disclosure is used as the food or feed additive, the peptide may be added as it is or used together with other foods, feeds, or ingredients thereof, and may be used appropriately according to conventional methods. The mixed amount of the active ingredient may be suitably determined according to the purpose of use (prevention, health, or therapeutic treatment of infectious diseases by inhibiting the growth and proliferation of bacteria). In general, when preparing feeds, foods or beverages, the peptide of the present disclosure is added in an amount of 15 wt% or less, preferably 10 wt% or less, based on a raw material. However, in the case of long-term intake for the purpose of health and hygiene or for the purpose of health control, the amount may be equal to or smaller than the range, and there is no problem in terms of safety, so that the active ingredients may be used even in an amount above the range. The types of food and feed are not particularly limited.

The present disclosure may have various modifications and various Examples, and specific Examples will be hereinafter illustrated in the drawings and described in detail in the detailed description. However, the present disclosure is not limited to specific Embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### [Examples]

### Example 1. Search for peptides consisting of transmembrane domain and total of three or more basic amino acids and some polar amino acids at opposite ends

In the present disclosure, transmembrane protein sequence information was searched from UniProt (www.uniprot.org), which contained information on protein sequences and functions. The UniProt homepage was accessed, the proteins UniProt knowledgebase was accessed, and then the reviewed (Swiss-Prot) was accessed. Next, when the proteins with transmembrane was accessed, 79,724 proteins were searched. For human proteins, 20,422 proteins were searched, of which 5,217 proteins were searched as transmembrane proteins. By accessing each transmembrane protein, information on each protein was checked and at the same time, peptide sequences of the transmembrane peptide domain could be checked. The present inventors first searched for a transmembrane domain and surrounding sequences in 5,217 transmembrane proteins, and synthesized a peptide consisting of a transmembrane domain with a structure similar to that in FIG. 1 and a total of three or more basic amino acids and some polar amino acids at opposite ends (Table 1).

### Example 2. Confirmation of antibacterial activity of peptides searched in UniProt

The present inventors synthesized 36 antibacterial peptides consisting of a transmembrane peptide domain searched in UniProt and basic or polar amino acids at opposite ends of the domain, and confirmed the antibacterial activity of each peptide using a minimum bactericidal concentration (MBC) measurement method (information on each peptide was shown in Table 1 below). The antibacterial activity of each peptide was confirmed using the MBC measurement method as follows.

First, *Pseudomonas aeruginosa* (ATCC 27853) and *Staphylococcus aureus* (NCCP 15872) were cultured overnight at 37°C and 200 rpm in an LB (1% tryptone, 0.5% yeast extract, 1% sodium chloride) liquid medium or a 3% (w/v) TSB (Tryptic Soy Broth) liquid medium, and then subcultured for 2 hours under the same conditions. The strain solution was prepared by diluting the subcultured strain with 10 mM sodium phosphate solution so that the final concentration of each strain was 5 × 10⁵ CFU/ml.

Next, 100 µl of each peptide was dispensed into each well of a 96-well microplate at different concentrations (final concentration: 0 µg/ml to 200 µg/ml peptide), 100 µl of the prepared strain solution was added, mixed, and reacted in an incubator at 37°C for 1 hour.

Then, 25 g/L of LB or 30 g/L of TS (Tryptic Soy) and 15 g/L of agar were dissolved in distilled water and sterilized, and then 20 mL of the mixture was poured into a 100 mm circular plate, and solidified at room temperature for one day or more to prepare a LB or TS agar plate. 10 µl of the reaction solution of the peptide and strain was spread on the prepared LB or TS agar plate to a predetermined size, and cultured in an incubator at 37°C for 18 hours, and then colony formation was confirmed on the plate. The MBC was defined as a minimum concentration of a peptide at which a single colony was not generated, and experimental results were shown in Table 1 below.

**[Table 1]**

| SE Q. No | Pept ide nam e | Uni Por t ent ry | Protein name | Gene name | Total amino acid number/anti bacterial peptide position | Peptide sequence (transmembr ane domain/amin o acid number) | Position in cell | MBC value (µg/ml) | | Num ber of amin o acids at opposite ends of trans mem brane doma in | |
|---|---|---|---|---|---|---|---|---|---|---|---|
| | | | | | | | | Ps eu do m on as ae ru gi no sa | Staph ylococ cus aureus | K + R | Hy dro phil ic ami no aci d |
| 1 | CO X20 -1 | Q5 RI1 5 | Cytochrome c oxidase assembly protein COX20, mitochondrial -Human | COX20 | 118/31-57 | RHSILYGS LGSVVAGF GHFLFTSRI RR (34- 51/27) | Mitoc hondri al membr ane | 4 | 10 | 4 | 4 |
| 2 | HIG 1A-1 | Q9 Y2 41 | HIG1 domain family member mitochondrial -Human | HIG1A | 93/22-51 | RKAKEAPF VPVGIAGF AAIVAYGL YKLKSR (26-46/30) | Mitoc hondri al membr ane | 3 | 11 | 6 | 1 |
| 3 | PIR T | P0 C8 51 | Phosphoinosit ide-interacting protein-Human | PIRT | 131/84-123 | KSLSILKM VGPGFLSL GLMMLVC GLVWVPII KKKQKHR QK (94- 114/40) | Cellul ar membr ane | 2 | 10 | 8 | 5 |
| 4 | HIG 1C | A8 M V8 1 | HIG1 domain family member 1C-Human | HIG1C | 97/21-51 | RKSRDSPF VPIGIAGF VTVVSCGL YKLKYRR (27-47/31) | Mitoc hondri al membr ane | 2 | 9 | 6 | 2 |
| 5 | HIG 2A-1 | Q9 B W7 2 | HIG1 domain family member 2A, mitochondrial -Human | HIG2A | 106/81-105 | TRIAAQGF TVAAILLG LAVTAMK SR (83- 103/25) | Mitoc hondri al membr ane | 11 | 20 | 2 | 2 |
| 6 | PAP PA | Q5 QF B9 | Protein PAPPAS-Human | PAPPA-AS1 | 102/7-38 | RKKHRGL FLTTVAAL PIWNPISEF VKWYKSH K (13-33/32) | ER membr ane | 7 | 20 | 6 | 3 |
| 7 | PLG RK T-1 | Q9 HB L7 | Plasminogen receptor-Human | PLGRK T | 147/50-76 | SREFLKYF GTFFGLAA ISLTAGAIK KK (53- 73/27) | Cellul ar membr ane | 7 | 80 | 4 | 1 |
| 8 | PG AP6 | Q9 HC N3 | Post-GPI attachment to proteins factor 6-Human | PGAP6 | 771/543-568 | QQRAATL LLTLSNLM FLAPIAVS VRR (546- 566/26) | Cellul ar membr ane, Lysos omal membr ane | 6 | 50 | 3 | 2 |
| 9 | OP A1-1 | O6 031 3 | Dynamin-like 120 kDa protein, mitochondrial -Human | OPA1 | 960/92-118 | RLATRLLK LRYLILGS AVGGGYT AKKT (97- 113/27) | Mitoc hondri al membr ane | 1 | 4 | 4 | 3 |
| 10 | TO MM 20 | Q1 538 8 | Mitochondrial import receptor subunit TOM20 homolog-Human | TOMM2 0 | 145/4-30) | RNSAIAAG VCGALFIG YCIYFDRK RRS (7- 24/27) | Mitoc hondri al membr ane | 20 | 35 | 5 | 3 |
| 11 | SLC 25A 19 | Q9 HC 21 | Mitochondrial thiamine pyrophosphat e carrier-Human | SLC25A 19 | 320(212-243) | KKNENLQ NLLCGSGA GVISKTLT YPLDLFKK R (220- 240/32) | Mitoc hondri al membr ane | 5 | 30 | 5 | 4 |
| 12 | VK OR L | Q8 N0 U8 | Vitamin K epoxide reductase complex subunit 1-like protein 1-Human | VKORC 1L1 | 176(13-42) | RWERVAR YAVCAAGI LLSIYAYH VEREKER (17-37/30) | ER membr ane | 30 | 25 | 5 | 0 |
| 13 | YM E1L 1 | Q9 6T A2 | ATP-dependent zinc metalloprotea se YME1L1-Human | YME1L 1 | 773(291-322) | RRTRLILF VLLLFGIY GLLKNPFL SVRFRTTT (296-316/32) | Mitoc hondri al membr ane | 6 | 18 | 5 | 4 |
| 14 | SLC 22A 14 | Q9 Y2 67 | Solute carrier family 22 member 14-Human | SLC22A 14 | 594(181-210) | TKKDTAQI MFMAGLPI GSLIFRLIT DKMGR (185-205/30) | Mitoc hondri al membr ane | 10 | 20 | 4 | 1 |
| 15 | SCO 2 | O4 381 9 | Protein SCO2 homolog, mitochondrial -Human | SCO2 | 266(58-82) | RTRLLITG LFGAGLG GAWLALR AEK (61- 78/25) | Mitoc hondri al membr ane | 16 | 25 | 4 | 1 |
| 16 | AB CB8 | Q9 NU T2 | Mitochondrial potassium channel ATP-binding subunit-Human | ABCB8 | 735(291-322) | STRLTLLL MVATPAL MGVGTLM GSGLRKLS RQ (296- 316/32) | Mitoc hondri al membr ane | 25 | 25 | 4 | 5 |
| 17 | UX S1 | Q8 NB Z7 | UDP-glucuronic acid decarboxylase 1-Human | UXS1 | 420(15-44) | RRRMKLL LGIALLAY VASVWGN FVNMRSIQ (20-40/30) | Golgi compl ex membr ane | 24 | 25 | 5 | 2 |
| 18 | TO MM 20L | Q6 UX N7 | TOMM20-like protein 1 | TOMM2 0L | 152(5-34) | RSLLRLLA AAAACGA FAFLGYCI YLNRKRR (10-29/30) | Mitoc hondri al membr ane | 16 | 10 | 6 | 2 |
| 19 | CN R1 | P2 155 4 | Cannabinoid receptor 1 | CNR1 | 472(373-405) | KLIKTVFA FCSMLCLL NSTVNPIIY ALRSKDLR (378-399/33) | Mitoc hondri al membr ane, Cellul ar membr ane | 12 | 24 | 5 | 2 |
| 20 | AR MC X3 | Q9 UH 62 | Armadillo repeat-containing X-linked protein 3 | ARMC X3 | 379(5-35) | RKVGWVT AGLVIGAG ACYCIYRL TRGRKQN K (7-29/31) | Mitoc hondri al membr ane | 10 | 22 | 5 | 2 |
| 21 | GD AP1 -1 | Q8 TB 36 | Ganglioside-induced differentiation -associated protein 1 | GDAP1 | 358(285-318) | KRKTFNK VLGHVNNI LISAVLPT AFRVAKK RAPK (292- 312/34) | Mitoc hondri al membr ane | 1 | 4 | 8 | 2 |
| 22 | GD AP1 -2 | Q8 TB 36 | Ganglioside-induced differentiation -associated protein 1 | GDAP1 | 358(313-343) | KKRAPKV LGTTLVVG LLAGVGY FAFMLFRK R (320- 340/31) | Mitoc hondri al membr ane | 4 | 6 | 7 | 0 |
| 23 | GP AT2 | Q6 NU I2 | Glycerol-3-phosphate acyltransferas e 2, mitochondrial | GPAT2 | 795(444-476) | RRLSCHVL SASVGSSA VMSTAIM ATLLLFKH QK (450- 472/33) | Mitoc hondri al membr ane | 4 | 13 | 4 | 5 |
| 24 | CDS 2 | O9 567 4 | Phosphatidate cytidylyltransf erase 2 | CDS2 | 445(160-191) | TM3.RILSK YHRFISFTL YLIGFCMF VLSLVKK HYR (166- 186/32) | ER membr ane | 50 | 45 | 5 | 2 |
| 25 | BCL 2L1 | Q0 781 7 | Bcl-2-like protein 1 | BCL2L1 | 233(204-233) | RKGQERF NRWFLTG MTVAGVV LLGSLFSR K (210- 22630) | Mitoc hondri al membr ane | 4 | 6 | 5 | 3 |
| 26 | ATP 5M K | Q9 6I X5 | ATP synthase membrane sub unit K, mitochondrial | ATP 5MK | 58(16-51) | KKYFNSYT LTGRMNC VLATYGSI ALIVLYFK LRSKK(23- 45/36) | Mitoc hondri al membr ane | 80 | 80 | 6 | 3 |
| 27 | CEB PZO S | A8 MT T3 | Protein CEBPZOS | CEBPZ OS | 80(10-38) | KKIFKGVL VAELVGV FGAYFLFS KMHTSQ(1 5-32/29) | Mitoc hondri al membr ane | 7 | 40 | 4 | 4 |
| 28 | EX D2 | Q9 NV H0 | Exonuclease 3'-5'domain-containing protein 2 | EXD2 | 621(2-32) | SRQNLVAL TVTTLLGV AVGGFVL WKGIQRR R(5-25/31) | Mitoc hondri al membr ane | 14 | 70 | 5 | 3 |
| 29 | MIG A1 | Q8 NA N2 | Mitoguardin 1 | MIGA1 | 632(64-96) | SQIKFSPV AKKLFVV TAVSAISVI FLAHFKRK R(70-90/33) | Mitoc hondri al membr ane | 5 | 7 | 5 | 4 |
| 30 | RH OT1 | Q8 IXI 2 | Mitochondrial Rho GTPase 1 | RHOT1 | 618(588-618) | KSSTFWLR ASFGATVF AVLGFAM YKALLKQ R(593- 615/31) | Mitoc hondri al membr ane | 8 | 5 | 3 | 4 |
| 31 | MT X1 | Q1 350 2 | Metaxin-1 | MTX1 | 466(416-445) | RRRNQILS VLAGLAA MVGYALL SGIVSIQR( 421-441/30) | Mitoc hondri al membr ane | 12 | 10 | 4 | 4 |
| 32 | MD UF A13 | Q9 P0J 0 | NADH dehydrogenas e [ubiquinone] 1 alpha subcomplex subunit 13 | NDUFA 13 | 144(27-58) | RRGLSGYS MLAIGIGT LIYGHWSI MKWNRER R(30-51/32) | Mitoc hondri al membr ane | 16 | 4 | 6 | 1 |
| 33 | SPN S1 | Q9 H2 V7 | Protein spinster homolog 1 | SPNS1 | 528(183-210 | QRSRMLSI FYFAIPVG SGLGYIAG SKVK(187- 207/28) | Mitoc hondri al membr ane | 4 | 20 | 4 | 2 |
| 34 | SLC 25A 44 | Q9 6H 78 | Solute carrier family 25 member 44 | SLC25A 44 | 314(17-49) | KKKFYVF GVAMTM MIRVSVYP FTLIRTRL QVQK(20- 42/33) | Mitoc hondri al membr ane | 8 | 3 | 5 | 3 |
| 35 | TM EM 14A | Q9 Y6 G1 | Transmembra ne protein 14A | TMEM1 4A | 99(21-51) | KRRGGVPS LIAGLFVG CLAGYGA YRVSNDK R(24-44/31) | Mitoc hondri al membr ane | 4 | 8 | 6 | 2 |
| 36 | IFI6 | P0 991 2 | Interferon alpha-inducible protein 6 | IFI6 | 130(2-27) | RQKAVSLF LCYLLLFT CSGVEAG KKK(4- 24/26) | Mitoc hondri al membr ane | 12 | 50 | 4 | 1 |

| | | | | | | | | | | | |
|---|---|---|---|---|---|---|---|---|---|---|---|
| (Underline: amino acid sequence of transmembrane domain) | | | | | | | | | | | |

As shown in Table 1 above, it was confirmed that 36 antibacterial peptides consisting of a transmembrane domain and basic or polar amino acids at opposite ends of the domain searched in UniProt had excellent antibacterial activity. The present inventors invented in previous studies that a peptide including a second transmembrane domain of a Romo1 protein had antibacterial activity, and invented in subsequent studies that a KU-5878 peptide consisting of 21 amino acid residues had the best antibacterial activity (MBC value of *Pseudomonas aeruginosa*: 100 µg/ml; MBC value of *Staphylococcus aureus*: 100 µg/ml). It was confirmed that the peptide discovered in the present disclosure had antibacterial activity that was 1.25 to 100 times better than KU-5878 against *Pseudomonas aeruginosa* and 1.25 to 25 times better than KU-5878 against *Staphylococcus aureus.*

### Example 3. Confirmation of antibacterial activity of peptides lacking some amino acids at opposite ends of transmembrane domain from peptides searched in UniProt

In order to confirm the antibacterial activity of peptides lacking some amino acids at opposite ends of a transmembrane domain in peptides searched in UniProt, a COX20-1 peptide was selected as a representative among the synthetic peptides shown in Table 1 above, and the antibacterial activity against *Pseudomonas aeruginosa* and *Staphylococcus aureus* was measured using the MBC measurement method. The MBC measurement method was the same as that of Example 2 above. Three peptides (SEQ ID NOs: 37, 38, and 39) including amino acid sequences lacking some amino acids at opposite ends of the COX20-1 peptide were synthesized, and the antibacterial activity of each peptide was compared and confirmed through the MBC measurement method (information on each peptide was shown in Table 2 below). Two peptides (SEQ ID NOs: 40 and 41) including amino acid sequences lacking some amino acids at opposite ends of a HIG1A-1 peptide were synthesized in the same experiment, and the antibacterial activity of each peptide was compared and confirmed through the MBC measurement method (information on each peptide was shown in Table 2 below).

**[Table 2]**

| SE Q. No | Pep tide na me | Uni Por t entr y | Protein name | Gene name | Total amino acid number/an tibacterial peptide position | Peptide sequence (transmembran e domain/amino acid number) | Positi on in cell | MBC value (µg/ml) | | Numbe r of amino acids at opposit e ends of transme mbrane domain | |
|---|---|---|---|---|---|---|---|---|---|---|---|
| | | | | | | | | Pseud omon as aerugi nosa | Staphy lococc us aureus | K + R | Hydr ophil ic amin o acid |
| 1 | CO X2 0-1 | Q5 RI1 5 | Cytochr ome c oxidase assembl y protein COX20, mitocho ndrial-Human | COX20 | 118/31-57 | RHSILYGSLG SVVAGFGHF LFTSRIRR (34-51/27) | Mitoc hondri al mem brane | 4 | 10 | 4 | 4 |
| 37 | CO X2 0-2 | Q5 RI1 5 | Cytochr ome c oxidase assembl y protein COX20, mitocho ndrial-Human | COX20 | 118/33-57 | SILYGSLGSV VAGFGHFLF TSRIRR (34- 51/25) | Mitoc hondri al memb rane | 30 | 55 | 3 | 3 |
| 38 | CO X2 0-3 | Q5 RI1 5 | Cytochr ome c oxidase assembl y protein COX20, mitocho ndrial-Human | COX20 | 118/31-54 | RHSILYGSLG SVVAGFGHF LFTSR (34- 51/24) | Mitoc hondri al memb rane | 25 | 200 < | 2 | 4 |
| 39 | CO X2 0-0 | Q5 RI1 5 | Cytochr ome c oxidase assembl y protein COX20, mitocho ndrial-Human | COX20 | 118/34-51 | ILYGSLGSV VAGFGHFLF (34-51/18) | Mitoc hondri al memb rane | 200 < | 200 < | 0 | 0 |
| 2 | HI G1 A-1 | Q9 Y2 41 | HIG1 domain family member 1A, mitocho ndrial-Human | HIG1A | 9/22-51 | RKAKEAPFV PVGIAGFAAI VAYGLYKL KSR (26- 46/30) | Mitoc hondri al memb rane | 3 | 11 | 6 | 1 |
| 40 | HI G1 A-2 | Q9 Y2 41 | HIG1 domain family member 1A, mitocho ndrial-Human | HIG1A | 93/23-49 | KAKEAPFVP VGIAGFAAI VAYGLYKL K (26-46/27) | Mitoc hondri al memb rane | 11 | 200 < | 4 | 0 |
| 41 | HI G1 A-0 | Q9 Y2 41 | HIG1 domain family member 1A, mitocho ndrial-Human | HIG1A | 93/26-46 | EAPFVPVGIA GFAAIVAYG LY (26-46/21) | Mitoc hondri al memb rane | 200 < | 200 < | 0 | 0 |

As shown in Table 2 above, it was confirmed that when some amino acids at opposite ends of the transmembrane domain were deficient, the antibacterial activity of the peptide decreased compared to a peptide that initially exhibited antibacterial activity (SEQ ID NO: 1 or 2). In particular, a peptide having no basic or polar amino acids at opposite ends of the transmembrane peptide domain had low antibacterial activity, making it impossible to be measured using the MBC measurement method (SEQ ID NOs: 39 and 41). Therefore, it may be seen that the basic amino acid and polar amino acid sequences at opposite ends of the transmembrane domain affect the antibacterial activity.

### Example 4. Confirmation of antibacterial activity of peptide synthesized by artificially attaching basic amino acids to opposite ends of transmembrane domain

In the present disclosure, it was confirmed that an antibacterial peptide group consisting of a transmembrane peptide domain and basic or polar amino acids at opposite ends of the domain exhibited antibacterial activity superior to that of KU-5878 invented in the previous study (Example 2 above). The antibacterial peptide group included peptides including amino acid sequences that existed in nature. In the example, peptides consisting of amino acid sequences that did not exist in nature and had similar characteristics to the antibacterial peptide group were synthesized, and the antibacterial activity of each peptide was compared and confirmed through a MBC measurement method (information on each peptide was shown in Table 3 below).

For KU-5878, the transmembrane domain corresponded to amino acids at positions 58 to 77. The transmembrane domain included an arginine (R) on the right side, but no basic amino acid on the left side. However, there was one basic amino acid, lysine (K) on the left side inside the transmembrane domain. In the present disclosure, the antibacterial activity of a peptide consisting of a total of three or more basic amino acids and some polar amino acids on opposite ends of the transmembrane domain was confirmed. As shown in Table 3, the antibacterial activity of synthesized peptides was measured by artificially attaching 1 to 5 lysines (K) or arginines (R) to the transmembrane domains of the HIG1A and COX20 proteins, respectively.

**[Table 3]**

| SE Q. No | Peptid e name | Uni Por t entr y | Protein name | Gen e nam e | Total amino acid number/ antibact erial peptide position | Peptide sequence (transmembrane domain/amino acid number) | Positi on in cell | MBC value (µg/ml) | | Numbe r of amino acids at opposit e ends of transm embran e domain | |
|---|---|---|---|---|---|---|---|---|---|---|---|
| | | | | | | | | Pseu domo nas aerug inosa | Staph yloco ccus aureus | K + R | Hyd roph ilic amin o acid |
| 41 | HIG1 A-0 | Q9 Y2 41 | HIG1 domain family member 1A, mitocho ndrial-Human | HIG 1A | 93/26-46 | EAPFVPVGIAGFA AIVAYGLY (26- 46/21) | Mitoc hondr ial mem brane | 200 < | 200 < | 0 | 0 |
| 42 | HIG1 A-0-K1 | - | - | - | - | KEAPFVPVGIAGF AAIVAYGLYK (23) | - | 200 < | 200 < | 2 | 0 |
| 43 | HIG1 A-0-K2 | - | - | - | - | KKEAPFVPVGIAG FAAIVAYGLYKK (25) | - | 18 | 100 | 4 | 0 |
| 44 | HIG1 A-0-K3 | - | - | - | - | KKKEAPFVPVGIA GFAAIVAYGLYKK K (27) | - | 2 | 2 | 6 | 0 |
| 45 | HIG1 A-0-K4 | - | - | - | - | KKKKEAPFVPVGI AGFAAIVAYGLYK KKK (29) | - | 1 | 2 | 8 | 0 |
| 46 | HIG1 A-0-K5 | - | - | - | - | KKKKKEAPFVPV GIAGFAAIVAYGL YKKKKK (31) | - | 1 | 2 | 1 | 0 |
| 47 | HIG1 A-0-R2 | - | - | - | - | RREAPFVPVGIAG FAAIVAYGLYRR (25) | - | 12 | 15 | 4 | 0 |
| 48 | HIG1 A-0-R3 | - | - | - | - | RRREAPFVPVGIA GFAAIVAYGLYRR R (27) | - | 5 | 2 | 6 | 0 |
| 49 | HIG1 A-0-R4 | - | - | - | - | RRRREAPFVPVGI AGFAAIVAYGLYR RRR (29) | - | 1 | 1 | 8 | 0 |
| 50 | COX2 0-0-K2 | - | - | - | - | KKILYGKLGKVV AGFGHFLFKK (22) | - | 7 | 18 | 4 | 0 |
| 51 | COX2 0-0-K3 | - | - | - | - | KKKILYGKLGKV VAGFGHFLFKKK (24) | - | 5 | 5 | 6 | 0 |
| 52 | COX2 0-0-R2 | - | - | - | - | RRILYGKLGKVVA GFGHFLFRR (22) | - | 5 | 10 | 4 | 0 |
| 53 | COX2 0-0-R3 | - | - | - | - | RRRILYGKLGKVV AGFGHFLFRRR (24) | - | 5 | 3 | 6 | 0 |
| 54 | SLC2 5A6-0 | P12 236 | ADP/A TP transloc ase 3 | SLC 25A 6 | 298/75-99 | LANVIRYFPTQAL NFAFKDKYKQIF (75-99/25) | Mitoc hondr ial mem brane | 200 < X | 200 < X | 0 | 0 |
| 55 | SLC2 5A6-0-K1 | - | - | - | - | KLANVIRYFPTQA LNFAFKDKYKQIF K (75-99/27) | - | 4 | 50 | 2 | 0 |
| 56 | SLC2 5A6-0-K2 | - | - | - | - | KKLANVIRYFPTQ ALNFAFKDKYKQI FKK (75-99/29) | - | 1 | 40 | 4 | 0 |
| 57 | SLC2 5A6-0-K3 | - | - | - | - | KKKLANVIRYFPT QALNFAFKDKYK QIFKKK (75-99/31) | - | 1 | 30 | 6 | 0 |
| 58 | ATP5 MC2 | Q0 605 5 | ATP synthase F(0) complex subunit C2, mitocho ndrial | AT P5 MC 2 | 141/80-104 | ATVGVAGSGAGIG TVFGSLIIGYAR (82-102/25) | Mitoc hondr ial mem brane | < 200 | < 200 | 1 | 1 |
| 59 | ATP5 MC2-0-K4 | - | - | - | - | KKKKVGVAGSGA GIGTVFGSLIIGYK KKK (29) | - | 4 | 8 | 8 | 0 |
| 60 | NDUF C2(N Dn-1) | O9 529 8 | NADH dehydro genase [ubiquin one] 1 subunit C2 | ND UF C2 | 119/54-77 | TAGLHRQLLYITA FFFAGYYLVKR (56-75/24) | Mitoc hondr ial mem brane | <200 | <200 | 2 | 1 |
| 61 | NDUF C2 (NDn-1)-0-K4 | - | - | - | - | KKKKGLHRQLLYI TAFFFAGYYLVKK KK (28) | - | 3 | 30 | 8 | 0 |
| 62 | HIGD 2B | Q4 VC 39 | Putative HIG1 domain family member 2B | HIG D2 B | 106/80-104 | HTQIAAQGFTIAAI LLGLAATAMKS (82-102/25) | Mitoc hondr ial mem brane | < 200 | < 200 | 1 | 3 |
| 63 | HIGD 2B-0-K4 | - | - | - | - | KKKKQIAAQGFTI AAILLGLAATAMK KKK (29) | - | 4 | 20 | 8 | 0 |
| 64 | ZDH2 3-K4-> ZDH2 3-0-K4 | Q8I YP 9 (T MD numb er) | Palmito yltransf erase ZDHHC 23 (TMD only) | | - | KKKKVGPVQLAV LTCGLFLILLALHK KKK (166-186/29) | - | 3 | 10 | 8 | 0 |
| 65 | PGAP 6-K4-> PGAP 6-0-K4 | Q9 HC N3 (T MD nu mb er) | Post-GPI attachm ent to proteins factor 6 (TMD only) | PG AP6 (TM D only ) | - | KKKKAATLLLTLS NLMFLAPIAVSVK KKK (546-566/29) | - | 1 | 30 | 8 | 0 |
| 66 | GHIT M-K4->GHI TM-0-K4 | Q9 H3 K2 (T MD nu mb er) | Growth hormon e-inducibl e transme mbrane protein (TMD only) | GHI TM (TM D only ) | - | KKKKSTYMYLAG SIGLTALSAIAISK KKK (126-146/29) | - | 3 | 6 | 8 | 0 |

As shown in Tables 2 and 3, peptides without basic or polar amino acids at opposite ends of the transmembrane domain had low antibacterial activity, making it impossible to be measured using the MBC measurement method. However, as confirmed in Table 3 above, it was found that a peptide group synthesized by artificially attaching two or more lysines or arginines exhibited antibacterial activity superior to KU-5878.

In addition, as a result of measuring antibacterial activity of peptides synthesized by further synthesizing transmembrane domains for SEQ ID NOs. 54, 58, 60, 62, 64, 65, and 66 and artificially attaching 1 to 4 lysines (K) to opposite ends of the domains through searching, it was found that the antibacterial activity of each peptide increased when lysines were artificially attached.

### Example 5. Confirmation of antibacterial activity of representative peptides among antibacterial peptides confirmed in Tables 1 and 3 against various bacteria

In order to confirm the types of bacteria against which the peptides with antibacterial activity confirmed in Examples 2 and 4 above exhibited bactericidal activity, three peptides OPA1-1, HIG1C, and COX20-1 among the synthetic peptides shown in Table 1 above and two peptides HIG1A-0-K4 and HIG1A-0-R4 among the synthetic peptides shown in Table 3 were selected as representative peptides, and antibacterial activity against the bacteria shown in Table 4 below was measured using the MBC measurement method. The MBC measurement method was the same as that of Example 2 above.

Specifically, each bacterium was cultured overnight in A 3% (w/v) TSB (Tryptic Soy Broth) liquid medium at 37°C and 200 rpm, and then subcultured again for 2 hours under the same conditions. The strain solution was prepared by diluting the subcultured strain with a 10 mM sodium phosphate solution so that the final concentration of each strain was 5 × 10⁵ CFU/ml.

Next, each peptide was dispensed into each well of a 96-well microplate at different concentrations (final concentration: 0 µg/ml to 200 µg/ml peptide), 100 µl of the prepared strain solution was added, mixed, and reacted in an incubator at 37°C for 1 hour.

Then, 30 g/L of TS (Tryptic Soy) and 15 g/L of agar were dissolved in distilled water and sterilized, and then 20 mL of the mixture was poured into a 100 mm circular plate, and solidified at room temperature for one day or more to prepare a TS agar plate. 10 µl of the reaction solution of the peptide and the strain was spread on the prepared TS agar plate to a predetermined size, and cultured in an incubator at 37°C for 18 hours, and then colony formation was confirmed on the plate. The MBC was defined as a minimum concentration of a peptide at which a single colony was not generated, and experimental results were shown in Table 4 below.

**[Table 4]**

| SEQ.No | Peptide name | *Enterococcus faecium* (14475) MBC value (µg/ml) | *E. coli* MBC. value (µg/ml) | *Acinetobacter baumannii* MBC value (µg/ml) |
|---|---|---|---|---|
| 1 | COX20-1 | 7 | 4 | 2 |
| 4 | HIG1C | 3 | 4 | 2 |
| 9 | OPA1-1 | 3 | 1 | 1 |
| 45 | HIG1A-0-K4 | 1 | 3 | 3 |
| 49 | HIG1A-0-R4 | 2 | 2 | 3 |

As may be confirmed in Table 4 above, it was found that antibacterial peptides consisting of a transmembrane peptide domain and basic or polar amino acids at opposite ends exhibited a wide range of antibacterial activity against both Gram-positive and Gram-negative bacteria.

### Example 6. Confirmation of antibacterial activity of representative peptides among antibacterial peptides confirmed in Tables 1 and 3 against multidrug-resistant bacteria

In order to confirm the antibacterial activity of the peptides confirmed in Examples 2 and 4 above against multidrug-resistant bacteria, three peptides OPA1-1, HIG1C, and COX20-1 among the synthetic peptides shown in Table 1 above and two peptides HIG1A-0-K4 and HIG1A-0-R4 among the synthetic peptides shown in Table 3 were selected as representative peptides, and antibacterial activity against the bacteria shown in Table 5 below was measured using the MBC measurement method. The MBC measurement method was the same as that of Example 2 above.

Meanwhile, in Table 5 below, multidrug-resistant *Pseudomonas aeruginosa* and multidrug-resistant Acinetobacter were bacteria isolated from patients at Korea University Anam Hospital. It was confirmed that the multidrug-resistant *Pseudomonas aeruginosa* was resistant to antibiotics such as piperacillin, piperacillin-tazobactam, ceftazidime, imipenem, meropenem, gentamicin, amikacin, and ciprofloxacin, and the multidrug-resistant Acinetobacter was resistant to antibiotics such as piperacillin, piperacillin-tazobactam, ceftazidime, imipenem, meropenem, gentamicin, amikacin, ciprofloxacin, and cefepime, and used in the experiment.

In addition, in Table 5 below, vancomycin-resistant *Enterococcus faecium* and vancomycin-resistant *Staphylococcus aureus* were purchased from the National Culture Collection for Pathogens (NCCP). It was confirmed that the vancomycin-resistant *Enterococcus faecium* was resistant to vancomycin, rifampin, tetracycline, gentamicin, erythromycin, streptomycin, and ampicillin, and the vancomycin-resistant *Staphylococcus aureus* was resistant to vancomycin, oxacillin, benzylpenicillin, ampicillin, and cefazolin, and used in the experiment.

**[Table 5]**

| SEQ.No | Peptide name | Multidrug-resistant *Pseudomonas aeruginosa* MBC value (µg/ml) | Multidrug-resistant *Staphylococcus aureus* (16870) MBC value (µg/ml) | Multidrug-resistant *Enterococcus faecium* (14475) MBC value (µg/ml) | Multidrug-resistant *Acinetobacter baumannii* MBC value (µg/ml) |
|---|---|---|---|---|---|
| 1 | COX20-1 | 6 | 18 | 7 | 4 |
| 4 | HIG1C | 2 | 8 | 2 | 2 |
| 9 | OPA1-1 | 0.5 | 6 | 2 | 1 |
| 45 | HIG1A-0-K4 | 3 | 2 | 1 | 3 |
| 49 | HIG1A-0-R4 | 2 | 2 | 2 | 2 |

As may be confirmed in Table 5 above, it was found that antibacterial peptides consisting of a transmembrane peptide domain and basic or polar amino acids at opposite ends of the domain exhibited a wide range of antibacterial activity against both multidrug-resistant Gram-positive bacteria and multidrug-resistant Gram-negative bacteria.

### Example 7. Confirmation of antibacterial activity of peptides consisting of transmembrane domain and total of three or more basic amino acids at opposite ends and some polar amino acids among proteins corresponding to other species in some peptides of antibacterial peptides in Table 1

The present inventors confirmed in Example 1 above that the peptide consisting of the transmembrane domain and the basic or polar amino acids at opposite ends of the domain in a human protein had excellent antibacterial activity. In order to confirm that a peptide of the same motif also had antibacterial activity among proteins of other species, peptides shown in Table 6 below were synthesized and antibacterial activity against *Pseudomonas aeruginosa* and *Staphylococcus aureus* was measured using a MBC measurement method. The MBC measurement method was the same as that of Example 2 above.

**[Table 6]**

| SE Q.N o | Pept ide nam e | Uni Port entr y | Protein name | Gene name | Peptide sequence (transmembran e domain/amino acid number) | Positio n in cell | MBC value (µg/ml) | | Number of amino acids at opposite ends of transme mbrane domain | |
|---|---|---|---|---|---|---|---|---|---|---|
| | | | | | | | Pseud omona s aerugi nosa | Staphyl ococcus aureus | K + R | Hydr ophili c amino acid |
| 67 | CO X-20-mou se | Q9D 7J4 | Cytochrom e c oxidase assembly protein COX20, mitochondri al | COX-20 | RESILYGSLG SIVTGLGHFL VTSRIRR (27) | Mitoch ondrial membr ane | 30 | 80 | 4 | 3 |
| 68 | OPA 1-rat | Q2T A68 | Dynamin-like 120 kDa protein, mitochondri al | OPA1 | RLAARLLKL RYIILGSAVG GGYTAKKT (97-113/27) | Mitoch ondrial membr ane | 2 | 2 | 4 | 2 |
| 69 | GD AP1 bovi ne | A6Q QZ0 | Ganglioside -induced differentiati on-associated protein 1 | GDAP1 | KRKTFNKVL GHVNNILISA VLPTAFRVA KKR (292- 312/31) | Mitoch ondrial membr ane | 4 | 4 | 7 | 2 |
| 70 | BCL 2L1-chic k | Q07 816 | Bcl-2-like protein 1 | BCL2L1 | RKGQETFNK WLLTGATVA GVLLLGSLL SRK (206- 223/30) | Mitoch ondrial membr ane | 8 | 8 | 4 | 4 |

As may be confirmed in Table 6 above, it was found that antibacterial peptides consisting of the transmembrane peptide domain and the basic or polar amino acids at opposite ends of the domain among the proteins corresponding to other species also exhibited antibacterial activity.

### Example 8. Confirmation of antibacterial activity of peptide synthesized by fusing transmembrane domains and N-terminal and C-terminal sequences at opposite ends of domains in two or more different antibacterial peptides

The present inventors produced a peptide by fusing transmembrane domains of SEQ ID NOs: 5 and 60 of the antibacterial peptide of the previous Example with amino acid sequences (KAK---KLK) at opposite ends of a domain of SEQ ID NO. 40, and then measured the antibacterial activity against *Pseudomonas aeruginosa* and *Staphylococcus aureus* using the MBC measurement method. The MBC measurement method was the same as that of Example 2 above. As a result, it was confirmed that the transmembrane domains of SEQ ID NOs. 5 and 60 had no antibacterial activity by themselves, but a new fusion peptide consisting of these transmembrane domains and four basic amino acids at opposite ends exhibited excellent antibacterial activity (SEQ ID NOs: 71 and 72).

**[Table 7]**

| SEQ .No | Pept ide nam e | UniPort entry of Transme mbrane domain | Protein name of transmembrane domain | Peptide sequence (transmembrane domain/amino acid number) | MBC value (µg/ml) | | Number of amino acids at opposite ends of transmem brane domain | |
|---|---|---|---|---|---|---|---|---|
| | | | | | Pseudo monas aerugin osa | Staphylo coccus aureus | K + R | Hydro philic amino acid |
| 71 | HI2 -m1 | Q9BW72 | HIG1 domain family member 2A, mitochondrial-Human | KAKIAAQGFTV AAILLGLAVTA MKKLK (27) | 3 | 12 | 4 | 0 |
| 72 | ND n-1-m1 | O95298 | NADH dehydrogenase [ubiquinone] 1 subunit C2 | KAKGLHRQLLY ITAFFFAGYYLV KLK (26) | 13 | 25 | 4 | 0 |

As may be confirmed in Table 7 above, it was found that the fusion peptides consisting of the transmembrane peptide domain and the basic or polar amino acids at opposite ends of the domain in the same pattern as Examples above all also exhibited excellent antibacterial activity.

Hereinabove, the present disclosure has been described with reference to preferred examples thereof. It will be understood to those skilled in the art that the present disclosure may be implemented as modified forms without departing from an essential characteristic of the present disclosure. Therefore, the disclosed examples should be considered in an illustrative viewpoint rather than a restrictive viewpoint. The scope of the present disclosure is illustrated by the appended claims rather than by the foregoing description, and all differences within the scope of equivalents thereof should be construed as being included in the present disclosure.

## Claims

1. A fusion polypeptide comprising a polypeptide including a transmembrane domain (TMD) consisting of 15 to 30 amino acids and terminal amino acids including one or more, a total of three or more amino acid bound to each of the C-terminus and the N-terminus of the polypeptide.

2. The fusion polypeptide of claim 1, wherein the transmembrane domain (TMD) is any one of SEQ ID NOs: 39, 41, 54, and 73 to 78.

3. The fusion polypeptide of claim 1, wherein the terminal amino acid includes 2 to 6 amino acids bound to each of the C-terminus and the N-terminus.

4. The fusion polypeptide of claim 1, wherein the terminal amino acid is selected from the group consisting of basic amino acids and polar amino acids.

5. The fusion polypeptide of claim 1, wherein the terminal amino acid is any one of lysine, arginine, histidine, serine, threonine cysteine, glutamine, asparagine and tyrosine.

6. The fusion polypeptide of claim 1, wherein the fusion polypeptide is any one of SEQ ID NOs: 43, 44, 45, 46, 47, 48, 49, 50, 51, 52, 53, 56, 57, 59, 61, 63, 64, 65, 66, 71 and 72.

7. An antibacterial polypeptide comprising the fusion polypeptide of claim 1.

8. The antibacterial polypeptide of claim 7, wherein the polypeptide further comprises PEGylation, acetylation, carboxylation, lipidation, or amidation at the N-terminus or C-terminus.

9. The antibacterial polypeptide of claim 7, wherein the polypeptide has antibacterial activity against one or more bacteria selected from the group consisting of Gram-positive bacteria, Gram-negative bacteria, and multidrug-resistant bacteria.

10. An antibiotic comprising the polypeptide according to any one of claims 1 to 9 as an active ingredient.

11. A food additive comprising the polypeptide according to any one of claims 1 to 9.

12. A feed additive comprising the polypeptide according to any one of claims 1 to 9.

13. A cosmetic composition comprising the polypeptide according to any one of claims 1 to 9.

14. An antibacterial biopesticide preparation comprising the polypeptide according to any one of claims 1 to 9.

15. An antibacterial quasi-drug composition comprising the polypeptide according to any one of claims 1 to 9.
